# EUROPÄISCHE PATENTANMELDUNG

(11) **EP 3 023 111 A1**
(43) Veröffentlichungstag der Anmeldung: **25.05.2016**
(21) Anmeldenummer: 15194852.8
(22) Anmeldetag: 17.11.2015
(51) Int. Cl.: A61M 1/00

(54) **FLÜSSIGKEITSINDIKATOR FÜR UNTERDRUCKTHERAPIE-VORRICHTUNG**

(30) Priorität: 19.11.2014 DE 102014116912
(71) Anmelder: Paul Hartmann AG, 89522 Heidenheim (DE)
(72) Erfinder: Croizat, Pierre, 89522 Heidenheim (DE); Deibler, Martin, 89522 Heidenheim (DE)

(57) **Zusammenfassung**

Die Erfindung betrifft eine am Körper eines Benutzers tragbare und/oder stationäre Vorrichtung zur Bereitstellung von Unterdruck für die Unterdrucktherapie von Wunden am menschlichen oder tierischen Körper. Die Vorrichtung umfasst eine den Unterdruck erzeugende Einrichtung, einen Anschluss für eine zur Wundstelle führende Saugleitung, eine Unterdruckverbindung, einen Filter sowie einen Flüssigkeitsindikator zum Anzeigen und/oder Nachweis von Flüssigkeit, welche stromabwärts des Filters (6) eingedrungen ist. Die Erfindung betrifft weiterhin die Verwendung eines Flüssigkeitsindikators im Zusammenhang mit einer Vorrichtung zur Bereitstellung von Unterdruck für die Unterdrucktherapie von Wunden.

## Beschreibung

Die Erfindung betrifft allgemein Vorrichtungen und Verfahren zur Bereitstellung von Unterdruck für medizinische Anwendungen, insbesondere Vorrichtungen zur Unterdrucktherapie von Wunden am menschlichen oder tierischen Körper und deren Verwendung. Die Vorrichtungen umfassen eine den Unterdruck erzeugende Einrichtung, einen Anschluss für eine Saugleitung, eine Unterdruckverbindung, einen Filter sowie einen Flüssigkeitsindikator.

Unterdrucktherapie von Wunden, im Fachbereich auch bezeichnet als TNP (topical negative pressure) oder NPWT (negative pressure wound therapy), ist seit langem bekannt, findet aber insbesondere seit Mitte/Ende der 1990er Jahre in zunehmendem Maße Anwendung. Dabei wird üblicherweise ein Wundfüllmaterial in die Wunde eingelegt, das Wundgebiet mit einer Folie abgedeckt und mittels eines Drainageschlauchs und einer Vakuumpumpe ein Unterdruck im Wundraum erzeugt. Durch die Verwendung des Wundfüllmaterials wird der Druck gleichmäßig über die Wunde verteilt. Der Unterdruck bewirkt, insbesondere in der initialen Phase der Wundbehandlung, eine effektive Wundreinigung durch Abtransport von Wundexsudat. Als weitere Vorteile werden, insbesondere in der nachfolgenden Granulationsphase, Förderung der Bildung von Granulationsgewebe und Verminderung der Wundödembildung genannt. Die Unterdrucktherapie wird üblicherweise bis zum Wundverschluss durchgeführt. Der Wundverschluss gilt im Allgemeinen als erreicht, wenn über der vormaligen Wundstelle eine Schicht Epidermis vorhanden ist. Ein Wundverschluss kann durch Heilungsprozesse oder durch chirurgische Eingriffe erreicht werden.

Unterdrucktherapie wird insbesondere eingesetzt bei akuten oder chronischen Wunden, beispielsweise bei Geschwüren (z.B. Druckgeschwüren, diabetischen, neuropathischen oder venös bedingten Geschwüren), traumatischen Wunden, therapieresistenten Wunden, infizierten Wunden, postoperativen Wunden, sondierten Fisteln sowie Hautlappen und Hauttransplantaten. Chronische Wunden sind durch eine verlangsamte oder fehlende Wundheilung gekennzeichnet.

Eine übliche Vorrichtung zur Unterdrucktherapie von Wunden umfasst eine den Unterdruck erzeugende Einrichtung (auch als "Unterdruckquelle" bezeichnet) und einen Anschluss für einen oder mehrere zur Wunde führende Saugleitungen. Häufig ist ein Flüssigkeitsaufnahmebehälter zur Aufnahme von aus einer Wunde abgesaugten Körperflüssigkeiten vorhanden.

Vorrichtungen zur Unterdrucktherapie von Wunden sind im Stand der Technik bekannt. Geeignete Unterdrucktherapiegeräte und Zubehör werden beispielsweise in den Dokumenten WO2011018132, WO2011018133, WO2012156140, WO2012156174, WO2012163617 und WO2013159904 beschrieben. Die Dokumente WO2014023501 und WO2011076340 offenbaren geeignete Unterdruckanschlussmittel (Port) zum Herstellen einer Unterdruckverbindung mit dem Wundverband. In den Wundraum können beispielsweise die aus den Dokumenten WO2010072309, WO2012022484, WO2011072840, WO2012167942 oder WO2011003521 bekannten Wundauflagen eingebracht werden. Als besonders vorteilhaft in der Unterdruck-Wundbehandlung hat sich nach der Erfahrung des Anmelders eine Wundauflage aus einem offenzelligen Polyester-Polyurethanschaum, die in der WO2012022485 näher beschrieben ist, erwiesen.

Geräte zur Unterdrucktherapie von Wunden sind im Stand der Technik beschrieben und in unterschiedlichsten Ausführungen kommerziell erhältlich. Die Mehrzahl der dem Markt erhältlichen Geräte verfügt über eine elektrisch antreibbare Pumpe zur Bereitstellung des Unterdrucks. Darüber hinaus wurden Vorrichtungen beschrieben, bei denen der Unterdruck manuell erzeugt wird, siehe beispielsweise das in der WO2009/103031 beschriebene Therapiesystem. Derartige Vorrichtungen, welche unabhängig von einer Stromversorgung eingesetzt werden können, sind mittlerweile gleichfalls kommerziell erhältlich. Das Spektrum der auf dem Markt verfügbaren Geräte umfasst kleine, tragbare Geräte, die den Patienten eine gewisse Mobilität ermöglichen bis hin zu stationär zu verwendenden Geräten, etwa in Langzeitpflegeeinrichtungen. Weiterhin sind Geräte verfügbar, wie beispielsweise in der WO2011018132 oder WO2011018133 beschrieben, die sich sowohl für den stationären als auch für den mobilen Betrieb eignen. Für die Unterdrucktherapie geeignetes Zubehör, wie beispielsweise Wundauflagen, weitere Verbandkomponenten oder Anschlussmittel ist in mannigfaltiger Ausgestaltung auf dem Markt verfügbar.

Der Ausdruck "Fluid" wird im vorliegenden Dokument als Sammelbegriff für Flüssigkeiten (beispielsweise Wasser, Spülflüssigkeiten oder Körperflüssigkeiten) und Gase (beispielsweise Luft oder Wasserdampf) verwendet. In den von der Wunde zum Therapiegerät führenden Leitungen (Saugleitung, Unterdruckverbindung) können Flüssigkeiten (insbesondere Körperflüssigkeiten), Gase (insbesondere Luft) oder Mischungen aus Flüssigkeiten und Luft vorliegen. Flüssigkeit kann auch in Form sehr feiner Tröpfchen in der zum Gerät hin gesaugten Luft vorhanden sein. Des Weiteren kann die Luft einen hohen Anteil an Wasserdampf enthalten (feuchte Luft).

Während der Unterdruckbehandlung werden Körperflüssigkeiten aus dem Wundbereich abgesaugt. Hierbei kann es sich insbesondere um Blut, Wundexsudat mit unterschiedlichen Wasseranteilen sowie um Spülflüssigkeiten handeln. Spülflüssigkeit fällt beispielsweise bei der so genannten Instillationstherapie an. Hierbei wird im Wechsel zur Beaufschlagung von Unterdruck eine die Wunde reinigende und/oder eine die Wundheilung fördernde Lösung in den Wundbereich appliziert. Die in der Unterdrucktherapie typischerweise anfallenden Flüssigkeitsvolumina hängen vom jeweils angewandten Behandlungsverfahren ab und können erheblichen Schwankungen unterliegen. Beim post-operativen Einsatz der Unterdrucktherapie zur Behandlung chirurgischer Wunden wird häufig nur eine geringe Menge frischen Blutes abgesaugt. Dagegen können beispielsweise bei einer Unterdrucktherapie am offenen Abdomen große Volumina (bis zu mehrere Liter pro Tag) von Körperflüssigkeiten mit einem hohen Wasseranteil anfallen.

In den aus dem Stand der Technik bekannten Vorrichtungen zur Unterdrucktherapie (siehe beispielsweise die oben erwähnte WO2011018132) sind häufig Flüssigkeitsaufnahmebehälter zur Aufnahme von aus einer Wunde abgesaugten Körperflüssigkeiten vorgesehen. Hierbei handelt es sich aus hygienischen Gründen meist um Behälter, welche nach einmaligem Gebrauch entsorgt werden (Wegwerfbehälter). Weiterhin gibt es auch Systeme zur Unterdruckbehandlung von Wunden, insbesondere zur Behandlung von Wunden mit geringem Exsudat-Aufkommen, bei denen kein Flüssigkeitsaufnahmebehälter vorgesehen ist. Das Wundexsudat wird bei derartigen Vorrichtungen dann meist im Wundverband gespeichert, wobei der Verband Flüssigkeits-absorbierende Komponenten aufweist, siehe beispielsweise die WO2009066105.

In jedem Fall stellt sich bei der Konstruktion einer Vorrichtung zur Unterdrucktherapie von Wunden das Problem, dass die abgesaugten Körperflüssigkeiten, welche häufig mit infektiösen Komponenten kontaminiert sind, nicht in die Vorrichtung zur Bereitstellung von Unterdruck (auch als "Unterdruckquelle" bezeichnet) gelangen dürfen. Der Eintritt von Körperflüssigkeiten in die Vorrichtung zur Bereitstellung von Unterdruck kann die Unterdruckquelle, also beispielsweise eine elektrisch angetriebene Pumpe oder eine manuell zu bedienende Unterdruckquelle, beschädigen. Dies kann zu einem sofortigen Ausfall der Unterdruckquelle führen, insbesondere wenn es sich um eine elektrisch angetriebene Pumpe handelt, so dass die Therapie unterbrochen und ein Ersatzgerät beschafft werden muss. Der Eintritt geringerer Mengen von Flüssigkeit kann zu einer Verkürzung der Lebensdauer der Unterdruckquelle führen. Weiterhin besteht Gefahr, dass die Flüssigkeit durch eine Unterdruckquelle hindurchgesaugt und über einen Auslass der Unterdruckquelle ausgeblasen wird. Hierbei können Gerätekomponenten beschädigt und mit infektiösen Flüssigkeiten kontaminiert werden. Im ungünstigsten Falle gelangt infizierte Flüssigkeit in die Umgebung. Eine Kontamination des Inneren des Gerätes oder der Umgebung kann geschehen, ohne dass der Benutzer dies bemerkt. Kontaminationen im Inneren einer elektrisch betriebenen Therapie-Vorrichtung können beispielsweise den Techniker, der das Gerät später wartet, gefährden. Kontaminationen in der Umgebung können den Patienten, das medizinische Personal ebenso wie andere Personen, die sich in der Umgebung des Therapiegerätes aufhalten, gefährden.

Es ist deshalb erforderlich, Flüssigkeitsanteile, welche sich in dem von der Unterdruckquelle angesaugten Luftstrom befinden, zuverlässig abzutrennen. Eine Separation von Luft und Flüssigkeit erfolgt üblicherweise bereits durch eine geeignete Konstruktion des Behälters, falls ein solcher vorgesehen ist. Wie oben erwähnt, ist es auch möglich, geringere Mengen an Körperflüssigkeiten in absorbierenden Lagen des Wundverbandes zurückzuhalten. Eine Separation von Luft und Flüssigkeit erfolgt in diesem Fall mittels der absorbierenden Lagen des Wundverbandes.

Körperflüssigkeiten können in die Unterdruckquelle insbesondere dann eindringen, wenn der Flüssigkeitsaufnahmebehälter oder eine andere Vorrichtung, welche zum Sammeln der abgesaugten Flüssigkeiten dient, wie beispielsweise eine absorbierende Verband-Lage, voll beziehungsweise gesättigt ist und die Unterdruckquelle nicht rechtzeitig abgeschaltet wird. Weiterhin können Flüssigkeiten in Form kleiner Tröpfchen vom Luftstrom mitgerissen werden und so in die Unterdruckquelle eindringen.

Die kommerziell erhältlichen Geräte enthalten deshalb normalerweise einen Filter in der Unterdruckleitung, welcher das Eindringen von Flüssigkeit in die Unterdruckquelle verhindern soll. Bei Geräten, welche einen Flüssigkeitsaufnahmebehälter aufweisen, befindet sich der Filter üblicherweise zwischen dem Behälter und dem Einlass der Unterdruckquelle. Häufig ist der Filter, bei dem es sich meist um einen Mikrofilter handelt, mit dem Behälter fest verbunden und wird beim Auswechseln des Behälters zusammen mit diesem entsorgt. Bei Geräten ohne Flüssigkeitsaufnahmebehälter, bei denen die Wundsekrete im Wundverband gespeichert werden, befindet sich der Filter meist in der Verbandkomponente, siehe die eingangs erwähnte WO2009/066105. Die Beschaffenheit des Filters ist derart, dass Luft hindurchtreten kann, Flüssigkeit jedoch zurückgehalten wird. Weiterhin ist bei üblichen elektrisch angetriebenen Unterdrucktherapie-Vorrichtungen häufig ein Mechanismus vorhanden, welcher dafür sorgt, dass das Gerät abgeschaltet wird, wenn der Behälter (falls vorhanden) voll ist. Im normalen Betrieb der Unterdrucktherapie-Vorrichtung können die oben genannten Filter, gegebenenfalls ergänzt durch einen Abschalt-Mechanismus bei vollem Behälter, den Eintritt von Flüssigkeit in die Pumpe verhindern.

Ein unerwünschter Flüssigkeitseintritt in die zur Unterdruckquelle hin führende Leitung und somit letztendlich in die den Unterdruck erzeugende Einrichtung kann jedoch auftreten, wenn der Filter defekt ist. Denkbar ist, dass ein Filter aufgrund eines Herstellungsfehlers bereits von Anfang an beim Anlegen von Unterdruck zumindest geringe Mengen an Flüssigkeit durchlässt. Ein Defekt kann jedoch auch bei einem anfangs intakten Filter im Laufe des Unterdruckbetriebes auftreten. Insbesondere ist denkbar, dass ein ursprünglich intakter Filter anfangs die eintretende Flüssigkeit zurückhält. Die vom Filter zurückgehaltene Flüssigkeit befeuchtet den Filter. Wenn der befeuchtete Filter dann längere Zeit einem anhaltenden Unterdruck ausgesetzt ist, dies wäre beispielsweise bei einer Störung des Abschalt-Mechanismus denkbar, kann der Filter "durchbrechen", so dass ein Eintritt von Flüssigkeit in die stromabwärts des Filters vorhandenen Abschnitte des Fluidsystems möglich ist.

Die an der vorliegenden Erfindung beteiligten Erfinder standen vor der Aufgabe, Unterdrucktherapie-Vorrichtungen zu verbessern. Insbesondere sollten die Betriebssicherheit und die Lebensdauer erhöht sowie eine Überwachung des ordnungsgemäßen Betriebes einschließlich der Wartung und Sicherheitsüberprüfung einer solchen Vorrichtung erleichtert werden. Weiterhin sollten derartige Therapie-Vorrichtungen besser vor einem unerwünschten Eintritt von Flüssigkeit in die den Unterdruck erzeugende Einrichtung geschützt werden.

Die Aufgaben wurden gelöst durch eine am Körper eines Benutzers tragbare und/oder stationäre Vorrichtung zur Bereitstellung von Unterdruck für die Unterdrucktherapie von Wunden am menschlichen oder tierischen Körper, umfassend eine den Unterdruck erzeugende Einrichtung mit einem Einlass zum Ansaugen von Luft und einem Auslass zum Ausblasen von Luft, einen Anschluss für eine zur Wundstelle führende Saugleitung, eine Unterdruckverbindung zwischen dem Einlass und dem Anschluss, so dass eine Unterdruckkommunikation zwischen der den Unterdruck erzeugenden Einrichtung und der zur Wundstelle führenden Saugleitung herstellbar ist, sowie mindestens einen Filter, welcher im intakten Zustand für Flüssigkeiten undurchlässig ist, wobei der Filter in der Unterdruckverbindung angeordnet ist. Die Vorrichtung ist erfindungsgemäß dadurch gekennzeichnet, dass die sie mindestens einen Flüssigkeitsindikator zum Anzeigen und/oder Nachweis von Flüssigkeit umfasst, wobei der mindestens eine Flüssigkeitsindikator stromabwärts des Filters angeordnet ist. Der Ausdruck "stromabwärts" ist hierbei auf den Fluidstrom bezogen, welcher bei der vorgesehenen Verwendung der Vorrichtung in der Unterdrucktherapie ausgehend von der Wunde in Richtung zur der den Unterdruck erzeugende Einrichtung (Unterdruckquelle) und weiter zum Auslass der Unterdruckquelle hin bewegt wird.

Das Vorsehen eines stromabwärts des Filters angeordneten Flüssigkeitsindikators kann die Betriebssicherheit und die Lebensdauer einer Unterdrucktherapievorrichtung erhöhen sowie die Wartung des Gerätes erleichtern.

Stromabwärts des Filters sind in dem Fluidsystem der Vorrichtung, ausgehend vom Filter, im Wesentlichen folgende Abschnitte vorhanden:
i) Unmittelbar stromabwärts des Filters befindet sich normalerweise der Abschnitt zwischen dem Filter und der Unterdruckquelle. Der Abschnitt zwischen dem Filter und dem Einlass der Unterdruckquelle wird üblicherweise durch ein Leitungsmittel gebildet, beispielsweise durch einen Silikonschlauch. Es wäre grundsätzlich auch möglich, den Filter unmittelbar an dem Einlass der Pumpe anzubringen. In diesem Falle ist dann zwischen dem Filter und dem Einlass der Unterdruckquelle kein Leitungsmittel vorhanden.
ii) Weiter stromabwärts ist die Unterdruckquelle, welche im Betrieb den Fluidstrom bewegen kann, vorhanden. Die Unterdruckquelle, beispielsweise eine elektrisch antreibbare Pumpe, umfasst einen Einlass zum Ansaugen von Luft, einen Innenraum, durch den der Fluidstrom hindurch bewegt wird sowie einen Auslass zum Ausblasen der Fluide. Der Innenraum kann bei Betätigung der Unterdruckquelle wechselseitig sowohl mit dem Einlass als auch mit dem Auslass kommunizieren.
iii) Der Auslass kann in eine Öffnung münden, so dass die Fluide unmittelbar in die Atmosphäre entlassen werden. Alternativ können stromabwärts des Auslasses weitere den Fluidstrom leitende Komponenten vorhanden sein. Der "Bereich des Auslasses der Pumpe" umfasst nach der hier verwendeten Begrifflichkeit dem Auslass der Unterdruckquelle nachgeschaltete Leitungsmittel oder sonstige Hohlräume, deren stromabwärts gelegene Öffnung dann in die Atmosphäre mündet. Wenn es sich bei der Unterdruckquelle um eine elektrisch antreibbare Pumpe handelt, umfasst der Bereich des Auslasses in der Praxis häufig Geräuschdämpfungselemente und/oder Geruchsabsorber.

An die zu einer Wundstelle führende Saugleitung kann ein Unterdruckverband angeschlossen werden, welcher üblicherweise mindestens eine Wundauflage sowie eine luftdichte Abdeckung (typischerweise eine Selbstklebefolie) umfasst, wobei die Abdeckung an der die Wunde umgebenden intakten Haut klebend fixiert wird. Bei der Wundauflage handelt es sich häufig um einen offenzelligen Schaumstoff, beispielsweise um den in der oben erwähnten WO2012022485 beschriebenen Polyester-Polyurethanschaum. Die Unterdruckkommunikation zwischen Saugleitung und Wundverband wird durch ein Unterdruck-Anschlussmittel (Port) hergestellt.

Bei der den Unterdruck erzeugenden Einrichtung kann es sich um eine manuell zu betätigende Saugvorrichtung oder vorzugsweise um eine elektrisch angetriebene beziehungsweise elektrisch antreibbare Pumpe handeln, insbesondere um eine Membranpumpe, welche durch eine elektronische Steuerungseinheit angesteuert und kontrolliert wird. Bei der elektronischen Steuerungseinheit, falls vorhanden, handelt es sich vorzugsweise um eine programmierbare Steuerungseinheit, welche über eine Schnittstelle, beispielsweise eine USB-Schnittstelle, mit einem externen elektronischen Gerät kommunizieren kann. Mittels der Kommunikationsverbindung mit dem externen Gerät, beispielsweise mit einem tragbaren Computer, kann die Steuerungseinheit programmiert werden oder es können im Rahmen einer Wartung auf der Steuerungseinheit gespeicherte Betriebsdaten abgefragt werden. Während der Durchführung der Unterdrucktherapie ist das externe elektronische Gerät normalerweise nicht an die Vorrichtung angeschlossen. Die elektronische Steuerungseinheit umfasst vorzugsweise Bedienungselemente, beispielsweise einen Touchscreen, mittels denen ein Benutzer die für das Therapieverfahren gewünschten Parameter (insbesondere Höhe und Dauer des anzulegenden Unterdruckes oder gegebenenfalls ein komplexes Therapieschema mit wechselnden Phasen höheren oder niedrigeren Unterdruckes) eingeben kann.

Die Unterdruckverbindung zwischen dem Einlass der den Unterdruck erzeugenden Einrichtung und dem Anschluss für die Saugleitung ermöglicht die Herstellung einer Unterdruckkommunikation zwischen Unterdruckquelle und Saugleitung. Bei der Unterdruckverbindung handelt es sich normalerweise um einen oder mehrere miteinander verbundene Leitungsmittelabschnitte aus einem Kunststoff. Die Unterdruckverbindung und/oder die zur Wunde führende Saugleitung sollte eine geeignete Länge aufweisen, so dass die Vorrichtung von der Wundstelle beabstandet bereitgestellt werden kann. Insofern die Vorrichtung über einen optional vorhandenen Flüssigkeitsaufnahmebehälter verfügt, kann das Behälterinnere gleichfalls eine Komponente der Unterdruckverbindung sein. In der Unterdruckverbindung ist ein Filter angeordnet, welcher durchlässig für die angesaugte Luft ist und gleichzeitig den Durchtritt von Flüssigkeit blockiert, um - jedenfalls im intakten Zustand - den Eintritt von Flüssigkeit in den stromabwärts des Filters angeordneten Abschnitt des Fluidsystems und somit in die Unterdruckquelle zu verhindern. Beispiele für geeignete Filter umfassen Membranfilter (beispielsweise aus Zellulose-Lagen) oder Filter aus einem porösen Festkörper (beispielsweise ein Keramikmaterial, ein Kunststoffmaterial, ein Metall oder ein Sinterkörper). Im Falle von Filtern auf Basis poröser Festkörper können in vorteilhafter Weise eine oder mehrere zusätzliche quellfähige Komponenten vorhanden sein (beispielsweise Carboxymethylcellulose), welche den Durchtritt von Fluid sperren, sobald der Filter durchnässt.

Erfindungsgemäß umfasst die Vorrichtung somit mindestens einen Flüssigkeitsindikator zum Nachweis von Flüssigkeit, welche in stromabwärts des Filters angeordnete Abschnitte des Fluidsystems eingedrungen ist.

Die Verwendung eines Flüssigkeitsindikators bietet den Vorteil, dass bereits eine geringe Menge stromabwärts des Filters eingedrungene Flüssigkeit mit bloßem Auge erkennbar ist. In der Vergangenheit eingedrungene Flüssigkeit kann mittels des Indikators auch dann noch nachgewiesen werden, wenn die Unterdruckverbindung bereits wieder trocken ist. Das Eindringen von Flüssigkeit in den zur Unterdruckquelle hinführenden Abschnitt der Unterdruckverbindung deutet auf einen schadhaften Filter hin.

Der Nachweis eines Flüssigkeitseintrittes in stromabwärts des Filters angeordnete Abschnitte des Fluidsystems mittels eines Indikators kann durch Vergleich des Indikatorelementes mit einer Farbtafel (Referenz) erfolgen. Bei der Referenz kann es sich beispielsweise um eine Karte handeln, auf der die Färbung des Indikators im trockenen beziehungsweise im nassen Zustand abgebildet ist, wobei gegebenenfalls zusätzlich Zwischenstufen vorhanden sind. Die Karte wird dann neben den an der Vorrichtung vorhandenen Indikator gehalten, so dass die Verfärbung oder die Veränderung der Farbintensität an dem Indikator durch Vergleich mit der Referenz ermittelt werden kann.

Um das Eindringen von Flüssigkeit in stromabwärts des Filters angeordnete Abschnitte des Fluidsystems nachweisen zu können, muss der Flüssigkeitsindikator in einem Abschnitt des Fluidsystems lokalisiert sein, welcher sich gleichfalls stromabwärts des Filters befindet. Der Indikator kann beispielsweise auf der Innenseite eines Leitungsmittels, welches die Unterdruckverbindung zwischen dem Filter und der Unterdruckquelle bildet, vorhanden sein. Um eine Untersuchung des Indikators mit bloßem Auge zu ermöglichen, muss der die Indikatorsubstanz aufweisende Leitungsmittelabschnitt für einen Benutzer gut zugänglich sein. Die Indikatorsubstanz kann als Beschichtung der Innenseite des Leitungsmittels vorliegen. Alternativ kann die Indikatorsubstanz an einen Träger gebunden vorliegen, welcher dann auf die Innenseite des Leitungsmittels aufgebracht wird. Es können hierzu auch kommerziell erhältliche, selbstklebende Indikatorstreifen verwendet werden.

Unter einem Flüssigkeitsindikator (auch als Feuchtigkeitsindikator bezeichnet) wird in diesem Zusammenhang eine Substanz verstanden, welche nach Aufnahme einer wässrigen Lösung einen Farbumschlag oder eine Farbveränderung zeigt. Bei der Farbveränderung kann es sich auch um eine Veränderung der Farbintensität oder um eine Veränderung einer Graustufe handeln. Bei der wässrigen Lösung kann es sich beispielsweise um Wundexsudat oder um Spülflüssigkeit handeln.

Flüssigkeitsindikatoren sind kommerziell erhältlich. Ein bekannter Feuchtigkeitsindikator ist beispielsweise ein mit Kobaltchlorid (Kobalt(II)-chlorid) versetztes Silicagel (auch als "Blaugel" bezeichnet), welches im trockenen Zustand eine blaue Farbe aufweist, die nach Wasseraufnahme nach violett beziehungsweise pink umschlägt. Der Farbumschlag ist abhängig von der Menge der aufgenommenen Flüssigkeit und von der Einwirkdauer der Flüssigkeit. Die Farbwechselreaktion beruht auf Wasseraufnahme. Daher spielt die Zeit der Einwirkung gleichfalls eine Rolle. Das Blaugel kann beispielsweise auf Zellulose als Trägermaterial aufgetragen werden. Da allerdings das im Blaugel enthaltene Kobaltchlorid als potenziell karzinogen eingestuft wird, sollten vorzugsweise Indikatorsubstanzen mit einem geringeren Gefährdungspotenzial verwendet werden. So kann beispielsweise Ammoniumeisen(III)-sulfat in Kieselgel eingesetzt werden. Dieser Indikator, welcher im trockenen Zustand orange gefärbt ist, entfärbt sich nach Wasseraufnahme zu blass-weiß.

Als Flüssigkeitsindikator eignet sich gleichfalls eine durch Wasser verteilbare Substanz, beispielsweise eine fluid-transportable Tinte und ein Trägermaterial, so dass nach Flüssigkeitsaufnahme eine Farbveränderung beobachtbar ist. Ein Klebeartikel mit einem Indikator, welcher eine durch Wasser verteilbare Substanz umfasst, ist beispielsweise in der WO2003/031531 beschrieben und unter der Bezeichnung "3M™ Water Contact Indicator Tape 5557" von der Firma 3M™ (USA) kommerziell erhältlich ("Indikator-Tape").

Der Flüssigkeitsindikator kann in demjenigen Abschnitt der Unterdruckverbindung, welcher sich zwischen dem Filter und dem Einlass befindet und/oder in der Unterdruck erzeugenden Einrichtung und/oder in dem Bereich des Auslasses vorhanden sein. Vorzugsweise umfasst die Vorrichtung einen Flüssigkeitsindikator, welcher zwischen dem Filter und dem Einlass vorhanden ist.

Gemäß einer besonders bevorzugten Ausführungsform ist der Flüssigkeitsindikator auf der zur Unterdruckquelle hin zeigenden Seite des Filters entweder an der Oberfläche des Filters oder in unmittelbarer Nähe zum Filter angebracht.

Gemäß einer weiteren vorteilhaften Ausbildung der Erfindung ist vorgesehen, den Farbumschlag oder die Farbveränderung an dem Indikator elektronisch zu detektieren. Dies kann mittels eines elektronischen Sensors erfolgen, welcher den Farbumschlag oder die Farbveränderung registrieren kann ("Farbsensor").

Die hier beschriebene Ausführungsform der Erfindung, welche die Verwendung eines Farbsensors vorsieht, kann in besonders vorteilhafter Weise bei einer Vorrichtung, welche eine elektrisch antreibbare Pumpe als Unterdruckquelle und eine elektronische Steuerungseinheit für die Pumpe umfasst, eingesetzt werden. Gemäß dieser besonders bevorzugten Ausführungsform umfasst die Vorrichtung einen elektronischen Sensor ("Farbsensor") zum Nachweis eines Farbumschlages oder einer Farbveränderung an dem Indikator, wobei der Farbsensor zwischen dem Filter und dem Einlass vorhanden ist, da hierdurch der Flüssigkeitseintritt bereits detektiert werden kann, bevor die Flüssigkeit in die Pumpe eingedrungen ist. Unter Verwendung der in dieser Ausführungsform beschriebenen Vorrichtung ist es nämlich möglich, die Pumpe unmittelbar nach einer Detektion von stromabwärts des Filters eingedrungener Flüssigkeit abzuschalten, bevor Flüssigkeit in die Pumpe eintritt. Die Erfindung umfasst somit auch eine Vorrichtung der hier beschriebenen Art, wobei die elektronische Steuerungseinheit die Pumpe unmittelbar nach einer Detektion von Flüssigkeit, welche stromabwärts des Filters eingedrungen ist, deaktivieren kann. Insbesondere kann hierdurch verhindert werden, dass eine größere Menge an Flüssigkeit durch die Pumpe hindurchgesaugt und über den Auslass aus dem geschlossenen Fluid-System ausgeblasen wird. Hierbei kann in vorteilhafter Weise die geringe Ansprechschwelle eines elektronischen Farbsensors und die hohe Verarbeitungsgeschwindigkeit einer elektronischen Steuerungseinheit ausgenutzt werden. Es ist daher möglich, nach einer Detektion von Flüssigkeit im angesaugten Luftstrom die Pumpe abzuschalten, bevor größere Mengen der Flüssigkeit in die Pumpe eintreten oder in die Umgebung ausgeblasen werden. Hierdurch wird eine Beschädigung der Pumpe ebenso wie eine Kontamination des Gerätes oder der Umgebung des Gerätes weitestgehend vermieden. Alternativ oder zusätzlich ist es möglich, beim Ansprechen des elektronischen Farbsensors ein akustisches und/oder visuelles Warnsignal auszugeben, um den Benutzer auf die Störung (bei der es sich dann meist um ein "Durchbrechen" des Filters handelt) hinzuweisen. Denkbar wäre es in diesem Zusammenhang, das Warnsignal über eine Daten-Fernkommunikation, beispielsweise über das Internet, weiterzugeben. Es wäre dann möglich, unter anderem das medizinische Pflegepersonal und/oder ein Servicezentrum des Geräteherstellers auf die Störung aufmerksam zu machen. Alternativ oder zusätzlich kann das Ansprechen des elektronischen Farbsensors in einem zur elektronischen Steuerungseinheit gehörenden elektronischen Speicher festgehalten werden. Neben dem Ansprechen des Sensors und gegebenenfalls der Intensität oder dem Verlauf der Sensorsignale sollte der Zeitpunkt des Ereignisses oder der Ereignisse registriert werden. Die gespeicherten Informationen zum Ansprechen des Sensors können dann beispielsweise im Zuge einer Wartung des Gerätes abgerufen werden. Der Wartungstechniker erhält somit Hinweise auf eine Fehlfunktion des Gerätes (insbesondere des Filters) in der Vergangenheit. Entsprechende Meldungen zum Flüssigkeitseintritt können dann beispielsweise zu einer Überprüfung oder einem Austausch der Pumpe Anlass geben. Weiterhin erhält der Techniker den Hinweis auf eine mögliche Kontamination des Geräteinneren durch Körperflüssigkeiten. Entsprechend könnte das Gerät dann beispielsweise desinfiziert oder vollständig außer Betrieb gesetzt werden. Bei der Auswahl der zu ergreifenden Maßnahmen kann auch die Anzahl und Art der Sensormeldungen herangezogen werden. So könnte es in einem gewissen Umfang möglich sein, über die Häufigkeit und Intensität der Sensorsignale einen Rückschluss auf die Menge an eingedrungener Flüssigkeit anzustellen. Entsprechend umfasst die Erfindung die Verwendung eines elektronischen Farbsensors in einer Unterdrucktherapie-Vorrichtung der gemäß dieser Ausführungsform beschriebenen Art zum Nachweis von Flüssigkeitseintritt in einen stromabwärts des Filters gelegenen Abschnitt des Fluidsystems im Rahmen einer Sicherheitsüberprüfung oder Wartung der Vorrichtung. Die Erfindung umfasst gleichfalls die Verwendung des elektronischen Farbsensors zum Schutz der Pumpe, des Gerätes und der Umwelt vor Kontamination. Die gespeicherten Meldungen könnten über eine Daten-Fernkommunikation verfügbar gemacht werden können, beispielsweise im Zusammenhang mit einer Fernwartung des Gerätes durch ein Servicezentrum des Geräteherstellers.

Gemäß einer weiteren besonders bevorzugten Ausführungsform der Erfindung führt der Nachweis von Flüssigkeit in einem stromabwärts des Filters angeordneten Abschnitt des Fluidsystems durch den elektronischen Farbsensor zum sofortigen Abschalten der Pumpe, zur Ausgabe eines Warnsignals sowie zur Sperrung der Unterdrucktherapieeinheit für den Benutzer. Die Sperrung der Unterdrucktherapieeinheit kann nur durch einen Servicetechniker des Geräteherstellers, nicht jedoch durch den Benutzer selbst aufgehoben werden. Hierbei handelt es sich um eine Sicherheitsmaßnahme, welche gewährleisten soll, dass ein möglicherweise mit Wundsekreten kontaminiertes Gerät nicht weiter benutzt und stattdessen fachkundig überprüft wird.

Insbesondere umfasst die Erfindung die Verwendung eines elektronischen Farbsensors, zur Sicherheitsabschaltung der Pumpe und/oder zur Sperrung der Unterdrucktherapieeinheit und/oder zur Alarmierung eines Benutzers und/oder zur Speicherung des Ereignisses in einem zur elektronischen Steuerungseinheit gehörenden elektronischen Speicher. Unter einer Sicherheitsabschaltung der Pumpe wird vorliegend eine unverzügliche Deaktivierung der Pumpe beim Nachweis von Flüssigkeit in einem stromabwärts des Filters vorhandenen Abschnitt des Fluidsystems verstanden. Der elektronische Farbsensors muss mit der Steuerungseinheit kommunizieren können, da bei dieser Ausführungsform die Erkennung eines Flüssigkeitseintritts in die stromabwärts des Filters angeordneten Abschnitte des Fluidsystems mittels der Steuerungseinheit anhand des Sensorsignals erfolgt. Die Kommunikation erfolgt hierbei bevorzugt durch eine direkte elektrische Verbindung. Die Kommunikation kann auch durch eine andersartige Verbindung realisiert werden, beispielsweise durch eine optische Verbindung (Glasfaserleiter) oder drahtlos mittels Funkverbindung. Auch beim Vorhandensein eines elektronischen Farbsensors kann der Nachweis des unerwünschten Eindringens von Flüssigkeit in die stromabwärts des Filters angeordneten Abschnitte des Fluidsystems zusätzlich mit bloßem Auge erkannt werden, wenn der Indikator im Inneren der Unterdruckverbindung derart angebracht ist, dass er durch den Farbsensor nicht verdeckt wird.

Im Rahmen der hier offenbarten Erfindung umfasst die Vorrichtung vorzugsweise einen Flüssigkeitsaufnahmebehälter zur Aufnahme von aus der Wunde abgesaugten Körperflüssigkeiten. Vorteilhafterweise ist der Flüssigkeitsaufnahmebehälter in der Unterdruckverbindung zwischen dem Filter und dem Anschluss für die zur Wundstelle führende Saugleitung angeordnet. Der Behälter weist dann mindestens einen Zulauf zum Einspeisen der Körperflüssigkeiten und mindestens einen Ausgang zur Beaufschlagung mit Unterdruck auf.

Der Flüssigkeitsaufnahmebehälter dient zur Aufnahme und Aufbewahrung der von der Wundstelle am Patienten abgesaugten Flüssigkeiten, beispielsweise Wundexsudat und/oder Spülflüssigkeit. Bei dem Flüssigkeitsaufnahmebehälter handelt es sich üblicherweise um einen Einwegartikel, welcher nach der Unterdrucktherapie entsorgt wird. Vorteilhafterweise werden herstellerseitig Flüssigkeitsaufnahmebehälter mit unterschiedlichen Kapazitäten bereitgestellt. Kleinere Behälter, beispielsweise mit einem Fassungsvermögen von 100 ml bis 400 ml, könnten für den mobilen Betrieb der Vorrichtung vorgesehen sein, während größere Behälter, beispielsweise mit einem Fassungsvermögen von 400 ml bis 2000 ml, dann für einen stationären Betrieb eingesetzt werden.

Im Bereich des Zulaufs zum Einspeisen der Körperflüssigkeiten kann der Anschluss für die zur Wundstelle führende Saugleitung angebracht sein. Alternativ ist es möglich, dass an dem Zulauf ein Leitungsmittel (Schlauch) vorhanden ist, an dessen zum Körper des Patienten weisenden Ende dann der Anschluss für die Saugleitung angebracht ist. In diesem Falle bildet das vorgenannte Leitungsmittel einen Teil der Unterdruckverbindung zwischen dem Anschluss und dem Einlass der Pumpe. Die Unterdruckverbindung umfasst in diesem Fall den Schlauch, das Innere des Behälters sowie den Fluidweg bis zum Einlass der Unterdruckquelle.

Der Filter kann gemäß einer möglichen Ausführungsform mit dem Behälterausgang über ein Leitungsmittel verbunden werden. Gemäß einer bevorzugten Ausführungsform ist der Filter jedoch unmittelbar in dem Bereich des Behälterausgangs, welcher zur Beaufschlagung mit Unterdruck dient, vorhanden. Insbesondere ist der Filter fest und unlösbar an dem Flüssigkeitsaufnahmebehälter befestigt, so dass der Filter nach einem Gebrauch des Behälters zusammen mit diesem entsorgt wird. Hierbei ist es besonders vorteilhaft, wenn der Flüssigkeitsindikator direkt auf der zur Unterdruckquelle hin zeigenden Oberfläche des Filters vorhanden ist. Hierzu können beispielsweise schmale Streifen eines Indikator-Tapes auf die Oberfläche des Filters aufgebracht werden. Das Indikator-Tape muss hierbei so aufgebracht werden, dass der Durchfluss des Luftstroms nicht wesentlich behindert wird. Die Streifen können beispielsweise randständig aufgebracht werden und sollten nur einen kleinen Anteil der Oberfläche (beispielsweise 5 % bis 20 % der Oberfläche) bedecken. Allerdings kann auf diese Weise nur ein Flüssigkeitseintritt nachgewiesen werden, welcher in derjenigen Unterdruckbehandlung aufgetreten ist, bei der der betreffende Behälter verwendet wurde. Vor dem letzten Behälter-Wechsel aufgetretene Ereignisse lassen sich dann nicht nachweisen. Es könnte deshalb in einem stromabwärts des Filters angeordneten Abschnitt des Fluidsystems ein zusätzlicher Indikator vorhanden sein, welcher bei einem Behälterwechsel nicht entsorgt wird.

Die erfindungsgemäße Vorrichtung kann insbesondere dann besonders vorteilhaft eingesetzt werden, wenn die abgesaugten Flüssigkeiten in dem Aufnahmebehälter in flüssiger Form bis zur Entsorgung aufbewahrt werden. Mit dem Ausdruck "Aufbewahrung in flüssiger Form" ist gemeint, dass in dem Behälter keine Eindickungsmittel (auch als "trapping agents" bezeichnet) vorhanden sind. Insbesondere werden die abgesaugten Flüssigkeiten nicht in eingedickter Form bis zur Entsorgung gespeichert.

Vorzugsweise weist der Flüssigkeitsaufnahmebehälter in seinem Inneren durch Rippen oder Vorsprünge gebildete Bereiche oder miteinander kommunizierende Kammern auf, so dass die im Inneren des Behälters gesammelten Körperflüssigkeiten bei einer Bewegung des Behälters am unkontrollierten Schwappen gehindert werden.

Es hat sich als besonders vorteilhaft erwiesen, wenn die Vorrichtung ein erstes Gehäuseteil mit einer den Unterdruck erzeugenden Einrichtung (Pumpe oder manuelle Unterdruckquelle) und ein zweites Gehäuseteil, welches den Flüssigkeitsaufnahmebehälter bildet und welches an dem ersten Gehäuseteil der Vorrichtung manuell befestigbar ist und von dem ersten Gehäuseteil der Vorrichtung manuell lösbar ist, umfasst. Vorteilhafterweise werden herstellerseitig die zweiten Gehäuseteile (Flüssigkeitsaufnahmebehälter) mit unterschiedlichen Kapazitäten bereitgestellt. Kleinere Behälter, beispielsweise mit einem Fassungsvermögen von 100 ml bis 400 ml, könnten für den mobilen Betrieb der Vorrichtung vorgesehen sein, während größere Behälter, beispielsweise mit einem Fassungsvermögen von 400 ml bis 2000 ml, dann für einen stationären Betrieb eingesetzt werden. Dasselbe erste Gehäuseteil kann dann je nach Bedarf mit unterschiedlichen zweiten Gehäuseteilen kombiniert werden, so dass die Vorrichtung sowohl für den mobilen Betrieb als auch für den stationären Betrieb, beispielsweise im Krankenhaus, einsetzbar ist.

Falls es sich bei der Vorrichtung um ein elektrisch betriebenes Gerät handelt, können in dem ersten Gehäuseteil neben der Pumpe unter anderem die Steuerungseinheit, der elektronische Farbsensor (falls vorhanden) und gegebenenfalls eine Stromversorgung untergebracht sein. Bei der Stromversorgung zum Betrieb von elektrischen oder elektronischen Komponenten (beispielsweise Pumpe, Farbsensor, Steuerungseinheit oder Bedienelemente) kann es sich insbesondere um ein Netzteil und/oder um einen Akku handeln. Weiterhin können an dem ersten Gehäuseteil Bedienelemente für das Gerät (Mittel zum Bedienen der Steuerungseinheit durch einen Benutzer), insbesondere ein Touchscreen-Display, vorhanden sein. Erfindungsgemäß ist es bevorzugt, wenn an dem oder in dem ersten Gehäuseteil sämtliche Bestandteile des Therapiegerätes untergebracht beziehungsweise vorhanden sind, welche für eine dauerhafte Benutzung vorgesehen sind. Dagegen umfasst das zweite Gehäuseteil vorzugsweise die zur einmaligen Benutzung vorgesehenen Komponenten der Therapievorrichtung, nämlich den Flüssigkeitsaufnahmebehälter sowie entweder den Anschluss für die Saugleitung oder ein zum Anschluss führendes Leitungsmittel.

Weiterhin umfasst das zweite Gehäuseteil vorzugsweise den Filter, so dass dieser zusammen mit dem Behälter entsorgt werden kann. Gemäß einer besonders bevorzugten Ausführungsform ist der Filter dabei unmittelbar an dem Ausgang des Flüssigkeitsaufnahmebehälters vorhanden.

Die Überprüfung, ob während des Betriebes der Vorrichtung Flüssigkeit in einen stromabwärts des Filters vorhandenen Abschnitt des Fluidsystems eingedrungen ist, wird insbesondere im Zuge einer Wartung der Vorrichtung durchgeführt. Die vorliegende Erfindung betrifft deshalb gleichfalls ein Wartungsverfahren zum Nachweis von Flüssigkeitseintritt in einen stromabwärts des Filters vorhandenen Abschnitt des Fluidsystems, umfassend
i) Bereitstellen einer Vorrichtung, wie vorstehend beschrieben,
ii) Öffnen des Gehäuses (falls vorhanden) der Vorrichtung, so dass der Flüssigkeitsindikator sichtbar ist,
iii) Überprüfen, ob an dem Flüssigkeitsindikator ein Farbumschlag oder eine Farbveränderung erkennbar ist, wobei der Farbumschlag beziehungsweise die Farbveränderung vorzugsweise entweder im Vergleich zu einer neuen beziehungsweise nicht benutzten Vorrichtung oder durch Abgleich mit einer Referenz zu bewerten ist,
iv) gegebenenfalls Dokumentation der Überprüfung.

Das Öffnen des Gehäuses kann entfallen, wenn das Gerät mit einem elektronischen Farbsensor, welcher einen Farbumschlag oder eine Farbveränderung an dem Flüssigkeitsindikator detektieren kann, ausgestattet ist und über eine Steuerungseinheit verfügt, welche die Sensorsignale elektronisch speichern kann. Anstelle einer Inaugenscheinnahme des Flüssigkeitsindikators lässt sich der Flüssigkeitseintritt in diesem Falle durch ein Abrufen des in der Steuerungseinheit vorhandenen Speicherinhalts erkennen.

Falls an dem Flüssigkeitsindikator ein Farbumschlag oder eine Farbveränderung erkennbar ist, muss von einem Flüssigkeitseintritt stromabwärts des Filters und somit auch in die Unterdruckquelle ausgegangen werden. Ein Flüssigkeitseintritt in einen stromabwärts des Filters angeordneten Abschnitt der Unterdruckverbindung ist gleichfalls ein Hinweis, dass der Filter bei einer oder mehrerer vorangehenden Verwendungen der Vorrichtungen nicht ordnungsgemäß funktioniert hat. Weiterhin besteht bei elektrisch betriebenen Geräten die Gefahr einer Beschädigung der Pumpe durch die eingedrungene Flüssigkeit. Im ungünstigsten Falle können Körperflüssigkeiten durch Filter und durch die Pumpe hindurch gesaugt und in das Gehäuse der Vorrichtung oder in die Umgebung ausgeblasen worden sein. Entsprechend schließen sich an das oben genannte Verfahren zum Nachweis von Flüssigkeitseintritt im Falle eines positiven Flüssigkeitsnachweises einer oder mehrerer der folgenden Schritte an:
- Austausch des Filters,
- Austausch oder Überprüfung des zur Unterdruckquelle hinführenden Abschnittes der Unterdruckverbindung,
- Austausch oder Überprüfung der Unterdruckquelle einschließlich des Auslasses,
- Überprüfung und ggf. Dekontamination des Gehäuseinnenraumes,
- Überprüfung und ggf. Dekontamination der Umgebung, in der die Vorrichtung eingesetzt war.

Die hier genannten Maßnahmen (Austausch von Geräteteilen, Überprüfungen und gegebenenfalls Dekontamination) sind gleichermaßen anzuwenden, wenn der Flüssigkeitseintritt durch einen elektronischen Sensor, welcher einen Farbumschlag oder eine Farbveränderung an dem Flüssigkeitsindikator erkennen kann, detektiert wurde.

Die vorliegende Erfindung betrifft weiterhin ein Verfahren zur Unterdrucktherapie von Wunden, umfassend die Schritte:
i) Bereitstellen einer erfindungsgemäßen Vorrichtung,
ii) Durchführen der Unterdrucktherapie,
iii) Überprüfen des Flüssigkeitsindikators,
iv) Im Falle eines Farbumschlages oder einer Farbveränderung an dem Flüssigkeitsindikator ein oder mehrere Wartungs- und/oder Sicherheitsmaßnahmen wie Austausch des Filters, Austausch des zur Unterdruckquelle hinführenden Abschnittes der Unterdruckverbindung, Austausch oder Überprüfung der Unterdruckquelle einschließlich des Auslasses, Überprüfung und ggf. Dekontamination des Gehäuseinnenraumes oder Überprüfung und ggf. Dekontamination der Umgebung, in der die Vorrichtung eingesetzt war.

Die hier beschriebene Vorrichtung kann zusätzlich einen Flüssigkeitsindikator, welcher im Inneren des Gehäuses der Vorrichtung, jedoch außerhalb des Fluidsystems vorhanden ist, vorsehen. Ein derartiger Indikator kann dem Wartungspersonal einen Hinweis geben, dass das Gerät unerwünscht mit Flüssigkeit in Berührung gekommen ist. Derartige Flüssigkeitsindikatoren sind aus dem Stand der Technik bekannt und werden beispielsweise bei Mobiltelefonen eingesetzt.

Weitere Einzelheiten, Merkmale und Vorteile der Erfindung ergeben sich aus den beigefügten Patentansprüchen, aus der zeichnerischen Darstellung und aus der nachfolgenden Beschreibung beispielhafter Ausführungsformen der Erfindung.

### Figuren

Figur 1 zeigt eine einfach ausgestaltete Ausführungsform der erfindungsgemäßen Vorrichtung
Figuren 2 bis 4 zeigen weitere Ausführungsformen der vorliegenden Erfindung In Figur 5 ist eine ein erstes und ein zweites Gehäuseteil umfassende Ausführungsform der erfindungsgemäßen Vorrichtung gezeigt

### Bezugszeichen

- 1: Unterdruckquelle
- 2: Einlass der Unterdruckquelle zum Ansaugen von Luft
- 3: Auslass der Unterdruckquelle zum Ausblasen von Luft
- 4: Anschluss für eine zur Wundstelle führende Saugleitung
- 5: Unterdruckverbindung
- 6: Filter
- 7: Elektronische Steuerungseinheit
- 8: Flüssigkeitsindikator
- 9: Flüssigkeitsaufnahmebehälter
- 10: Vorrichtung zur Bereitstellung von Unterdruck
- 11: Verbindung zwischen Unterdruckquelle und Steuerungseinheit
- 12: Verbindung zwischen elektronischem Farbsensor und Steuerungseinheit
- 14: Schalldämpfer
- 15: Wundstelle
- 16: Luftundurchlässige Wundabdeckung
- 17: Unterdruck-Anschlussstück (Port)
- 18: Saugleitung
- 19: Elektronischer Farbsensor
- 20: An einen Unterdruckverband angeschlossene Vorrichtung zur Bereitstellung von Unterdruck
- 21: Elektrisch angetriebene Pumpe
- 22: Stromversorgung
- 23: Touchscreen-Display
- 24: Verbindungsmittel
- 25: Von der Wunde abgesaugte Körperflüssigkeiten
- 30: Eine ein erstes Gehäuseteil und ein zweites Gehäuseteil umfassende Vorrichtung
- 31: Erstes Gehäuseteil
- 32: Zweites Gehäuseteil

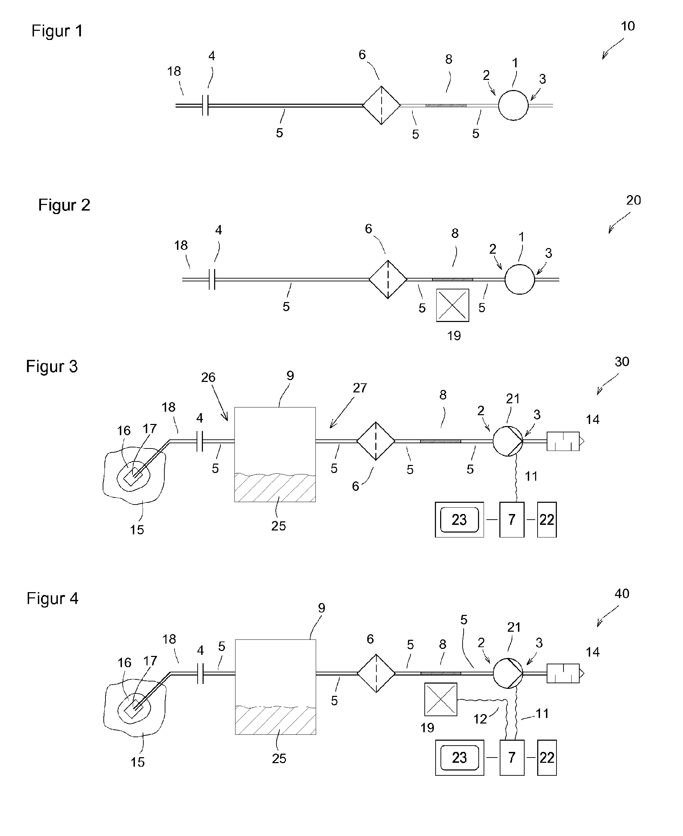

**Figur 1** zeigt in schematischer Darstellung eine erfindungsgemäße Vorrichtung 10 zur Bereitstellung von Unterdruck. Die Vorrichtung umfasst eine Unterdruckquelle 1 mit einem Einlass 2 zum Ansaugen von Luft und einem Auslass 3 zum Ausblasen von Luft, insbesondere eine elektrisch angetriebene beziehungsweise elektrisch antreibbare Pumpe. Weiterhin umfasst die Vorrichtung einen Anschluss 4, einen Filter 6, ein Flüssigkeitsindikator 8 sowie eine Unterdruckverbindung 5. Der Flüssigkeitsindikator 8 ist im Inneren desjenigen Abschnittes der Unterdruckverbindung 5 vorhanden, welche den Filter 6 mit dem Einlass 2 der Unterdruckquelle 1 verbindet.

Eine Unterdruckkommunikation zwischen Wundstelle (in Figur 1 nicht dargestellt) und Unterdruckquelle 1 kann über den Anschluss 4 hergestellt werden. Beim Anschluss 4 handelt es sich beispielsweise um einen üblichen Schnelltrennverbinder für Kunststoffschläuche. Zum Herstellen einer Unterdruck-Kommunikation wird eine Saugleitung 18 über den Anschluss 4 mit der Unterdruckverbindung 5 verbunden. Die Unterdruckverbindung 5, welche eine Unterdruckkommunikation zwischen dem Einlass 2 der Unterdruckquelle und dem Anschluss 4 ermöglicht, besteht bei der in Figur 1 gezeigten Ausführungsform aus zwei Abschnitten, nämlich dem Abschnitt zwischen Anschluss 4 und dem Filter 6 sowie dem Abschnitt zwischen Filter 6 und Unterdruckquelle 1. Die Unterdruckverbindung einer erfindungsgemäßen Vorrichtung kann weitere Abschnitte umfassen, siehe beispielsweise Figur 3. Die einzelnen Abschnitte der Unterdruckverbindung können Leitungsmittel, beispielsweise KunststoffSchläuche, umfassen. Es ist auch möglich, einen oder mehrere der genannten Komponenten in einem einzigen Bauteil zusammenzufassen. Beispielsweise können der Anschluss 4 und der Filter 6 in einem einzigen Bauteil verwirklicht werden, so dass zwischen diesen Elementen keine Leitungsmittel vorhanden sein müssen.

Bei der in **Figur 2** schematisch gezeigten Vorrichtung 20 zur Bereitstellung von Unterdruck umfasst die Vorrichtung zusätzlich einen elektronischen Farbsensor 19, welcher derart angebracht ist, dass er eine an dem Indikator auftretende Änderung der Farbintensität oder ein Farbumschlag detektieren kann. Dies kann beispielsweise dadurch erfolgen, dass eine im Inneren des den Filter 6 mit dem Einlass 2 der Unterdruckquelle 1 verbindenden Leitungsmittels 5 als Beschichtung aufgebrachte Indikatorsubstanz vorhanden ist, wobei das Leitungsmittel transparent sein muss. Der elektronische Farbsensor 19 befindet sich derart auf der Außenseite des Leitungsmittels, dass eine Farbveränderung im Inneren der Leitung durch den Sensor detektierbar ist. Es wäre auch möglich in den entsprechenden Abschnitt der Unterdruckverbindung 5, also zwischen dem Filter 6 und dem Einlass 2 der Unterdruckquelle 1 eine speziell angefertigte Komponente zwischenzuschalten, bei dem ein mit Indikatorsubstanz beschichtetes Leitungsmittel und der Farbsensor als integriertes Bauelement vorliegen. Ein Signal des Farbsensors 19, welches durch einen Farbumschlag an dem Indikator 8 ausgelöst wird, kann zur Ausgabe eines Warnsignals, zum Abschalten der Unterdruckquelle 1 oder zur Sperrung der Vorrichtung für den Benutzer herangezogen werden. Es ist auch möglich, das Signal zu speichern und/oder über eine Datenfernübertragung beispielsweise an ein Servicezentrum weiterzuleiten. Die Ausgabe eines Warnsignals, das Abschalten der Unterdruckquelle 1 oder die Sperrung der Vorrichtung kann über eine elektronische Schaltung realisiert werden. Vorzugsweise wird der Sensoroutput durch eine elektronische Steuerungseinheit (in Figur 2 nicht dargestellt, siehe Figur 4) erfasst, wobei die Steuerungseinheit eine oder mehrere der oben genannten Maßnahmen einleiten kann.

**Figur 3** zeigt eine weitere Ausführungsform der erfindungsgemäßen Vorrichtung in schematischer Darstellung, wobei die mit Bezugszeichen 30 bezeichnete Vorrichtung über den Anschluss 4 an einen Unterdruckverband angeschlossen ist. Der Unterdruckverband umfasst eine luftundurchlässige Wundabdeckung 16, ein Unterdruckanschlussstück (Port) 17 sowie die Saugleitung 18. Der Unterdruckverband wurde an einer Wundstelle 15 angelegt, wobei die Wundstelle 15 die zu behandelnde Wunde und die die Wunde umgebende intakte Haut des Patienten umfasst. Der Unterdruck wird in dieser Ausführungsform durch eine elektrisch angetriebene beziehungsweise elektrisch antreibbare Pumpe 21 zur Verfügung gestellt, die somit die Unterdruckquelle darstellt. Zusätzlich zu den in Figuren 1 und 2 gezeigten Ausführungsformen umfasst die in Figur 3 dargestellte Vorrichtung einen Flüssigkeitsaufnahmebehälter 9, in dem die von der Wunde des Patienten abgesaugten Körperflüssigkeiten 25, vorzugsweise in flüssiger Form, gesammelt werden. Im Inneren des Flüssigkeitsaufnahmebehälters 9 können durch Rippen oder Vorsprünge gebildete Bereiche oder miteinander kommunizierende Kammern vorhanden sein, so dass die gesammelten Körperflüssigkeiten bei einer Bewegung des Behälters am unkontrollierten Schwappen gehindert werden (in Figur 3 nicht dargestellt).

Der Flüssigkeitsaufnahmebehälter 9 weist einen Zulauf 26 zum Einspeisen der Körperflüssigkeiten und einen Ausgang 27 zur Beaufschlagung des Behälters mit Unterdruck auf. Entsprechend umfasst die Unterdruckverbindung 5 bei der in Figur 3 dargestellten Ausführungsform einen weiteren Abschnitt, nämlich das Innere des Flüssigkeitsaufnahmebehälters 9. Der Ausgang 27 ist in dem oberen Bereich des Flüssigkeitsaufnahmebehälters 9 derart angeordnet, dass normalerweise keine Flüssigkeiten 25 in den stromabwärts gelegenen Anteil der Unterdruckverbindung 5 gelangen, solange der Flüssigkeitsspiegel der abgesaugten Körperflüssigkeiten 25 den Ausgang 27 nicht erreicht. Körperflüssigkeiten könnten jedoch in den zur Pumpe 21 hinführenden Abschnitt der Unterdruckverbindung 5 eindringen, sobald der Flüssigkeitsspiegel das Niveau des Ausgangs 27 erreicht. Um ein Einsaugen von Flüssigkeit in die Pumpe zu verhindern, ist deshalb zwischen Behälter 9 und der Pumpe 21, wie im Stand der Technik üblich, ein Filter 6 vorhanden. Der Filter 6 lässt im intakten Zustand keine Flüssigkeit in den zur Pumpe hinführenden Abschnitt der Unterdruckverbindung 5 eindringen. Um im Falle eines defekten Filters 6 in Richtung Pumpe 21 gesaugte Flüssigkeit nachweisen zu können, ist zwischen dem Filter 6 und dem Einlass 2 der Pumpe 21 ein Flüssigkeitsindikator 8 vorgesehen. Weiterhin umfasst die Vorrichtung eine elektronische Steuerungseinheit 7, welche mit der Pumpe 21 über die Verbindung 11 kommunizieren und diese dadurch steuern kann. Bei der Verbindung 11 kann es sich um eine elektrische Verbindung oder um eine drahtlose Verbindung handeln. Die elektronische Steuerungseinheiten 7 kann mit Bedienungselementen, insbesondere mit einem Touchscreen-Display 23 verbunden sein. Der Benutzer des Gerätes kann die Therapievorrichtung 30 über das Touchscreen-Display 23 steuern und gegebenenfalls programmieren. Zusätzlich kann die elektronische Steuerungseinheit Schnittstellen (beispielsweise einen USB Anschluss) aufweisen, so dass externe elektronische Komponenten, beispielsweise ein tragbarer Computer, mit der Steuerungseinheit 7 kommunizieren können. Die Schnittstellen sind in Figur 3 nicht dargestellt. Bei der Komponente 22 handelt es sich um eine Stromversorgung, welche normalerweise einen aufladbaren Akkumulator für den mobilen Betrieb des Gerätes sowie ein Netzteil umfasst. Mittels der Stromversorgung 22 kann die elektrisch angetriebene Pumpe 21, die elektronische Steuerungseinheit 7 sowie weitere elektronische oder elektrische Komponenten wie beispielsweise das Touchscreen-Display 23 mit Strom versorgt werden.

In Figur 3 ist ein optional vorhandener Schalldämpfer 14 dargestellt, welcher an den Auslass 3 der Pumpe 21 angeschlossen ist, um die Betriebsgeräusche der Vorrichtung zu reduzieren. Der Schalldämpfer 14 kann geruchsabsorbierende Substanzen, beispielsweise Aktivkohle, enthalten, um eine Geruchsbelästigung beim Betrieb des Gerätes zu vermeiden.

Bei dem in Figur 3 dargestellten Beispiel erstreckt sich das Fluidsystem der Vorrichtung vom Anschluss 4 bis zur stromabwärts gelegenen Öffnung des Schalldämpfers 14, welche mit der Atmosphäre kommuniziert. Das Fluidsystem der Vorrichtung 30 kann wiederum mit dem Fluidsystem der patientenseitigen Komponenten (16, 17, 18) kommunizieren, wenn der Unterdruckverband über den Anschluss 4 mit der Therapievorrichtung dichtend verbunden wird. Entsprechend ist dann zwischen dem Wundraum des Patienten und der Öffnung des Schalldämpfers 14 ein zusammenhängendes Fluidsystem hergestellt, wobei in dem Abschnitt zwischen Wundraum und Pumpe 21 bei Betrieb der Pumpe 21 ein gegenüber der umgebenden Atmosphäre erniedrigter Luftdruck (Unterdruck) vorhanden ist. Fluide (insbesondere Wundsekrete und Luft) werden dann bei der vorgesehenen Verwendung der Vorrichtung in der Unterdrucktherapie ausgehend von der Wunde stromabwärts in Richtung zum Flüssigkeitsaufnahmebehälter 9 gesaugt.

Bei der in **Figur 4** gezeigten Vorrichtung 40 ist zusätzlich an dem Flüssigkeitsindikator 8 ein Farbsensor 19 vorhanden, welcher mit der elektronischen Steuerungseinheit 7 kommunizieren kann. Der Flüssigkeitsindikator 8 befindet sich im Inneren der Leitung, während der Farbsensor 19 außen an dem transparent ausgebildeten Leitung (beispielsweise ein Silikonschlauch) angebracht ist. Die elektronische Steuerungseinheit 7, welche mit dem elektronischen Farbsensor 19 über die Verbindung 12 (elektrisch oder drahtlos) kommuniziert, wertet den Sensoroutput von Farbsensor 19 aus, um das Eindringen von Flüssigkeit in den betreffenden Leitungsabschnitt detektieren zu können. Weiterhin kann die elektronische Steuerungseinheit 7 über die Verbindung 11 die Pumpe 21 (elektrisch oder drahtlos) steuern. Im Falle einer Detektion von Flüssigkeit durch den Farbsensor 19 kann die elektronische Steuerungseinheit 7 die Pumpe 21 deaktivieren, um ein Einsaugen von Flüssigkeit in die Pumpe 21 und ein Ausblasen von Körperflüssigkeiten über den Auslass 3 in das Geräteinnere (und gegebenenfalls zusätzlich in die Umgebung des Gerätes) zu verhindern. Weiterhin kann die elektronische Steuerungseinheit 7 die Vorrichtung für den Benutzer sperren. Alternativ oder zusätzlich kann die Steuerungseinheit 7 ein Warnsignal (optisch und/oder akustisch) ausgeben, um den Benutzer auf den defekten Filter 6 hinzuweisen. Weiterhin kann das detektierte Ereignis in einem zur elektronischen Steuerungseinheit 7 gehörenden Speicher (in Figur 4 nicht dargestellt) festgehalten werden.

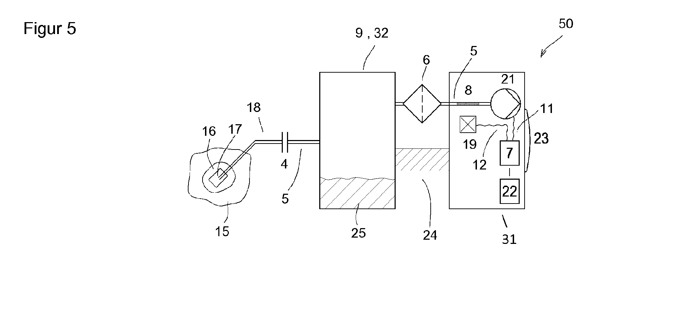

**Figur 5** zeigt in schematischer Darstellung eine weitere bevorzugte Ausführungsform der Vorrichtung 50 mit einem ersten Gehäuseteil 31 und einem zweiten Gehäuseteil 32. Das erste Gehäuseteil 31 umfasst neben der den Unterdruck erzeugenden Pumpe 21 die üblicherweise zum mehrmaligen Gebrauch vorgesehenen Komponenten, also insbesondere die elektronische Steuerungseinheit 7, den Farbsensor 19, die Stromversorgung 22 und das Touchscreen-Display 23. Somit können die zum einmaligen Gebrauch vorgesehenen Komponenten der Vorrichtung, nämlich Flüssigkeitsaufnahmebehälter 9 mit Filter 6 sowie Anschluss 4, von den zum mehrmaligen Gebrauch vorgesehenen weiteren Komponenten abgekoppelt werden. Normalerweise umfasst das erste Gehäuseteil 31 ein Gehäuse, in dem oder an dem die vorgenannten Komponenten untergebracht sind. Das zweite Gehäuseteil 32 bildet den Flüssigkeitsaufnahmebehälter 9 und kann weitere Komponenten, beispielsweise den Anschluss 4 oder alternativ eine zum Anschluss 4 führenden Leitung umfassen. Das zweite Gehäuseteil 32 ist an dem ersten Gehäuseteil 31 der Vorrichtung manuell befestigbar und von dem ersten Gehäuseteil 31 der Vorrichtung manuell lösbar. Die lösbare Verbindung der beiden Gehäuseteile (31,32) wird vorzugsweise unter Verwendung eines Kopplungsmittels 24 bewerkstelligt. Hierbei kann es sich beispielsweise um eine einschnappende Verriegelung handeln. Wie in Figur 5 gezeigt, ist der Filter 6 vorzugsweise unmittelbar an dem Ausgang 27 des Flüssigkeitsaufnahmebehälters 9 angebracht. Der Filter 6 sollte hierbei auf seiner zur Pumpe 1 hin zeigenden Seite ein zum Herstellen einer druckdichten Verbindung geeignetes Kopplungselement aufweisen, beispielsweise eine ringförmige Dichtung, so dass beim Aneinanderfügen der Gehäuseteile gleichzeitig eine Unterdruckkommunikation zwischen Behälter und dem pumpenseitigen Abschnitt der Unterdruckverbindung 5 hergestellt werden kann.

## Patentansprüche

1. Am Körper eines Benutzers tragbare und/oder stationäre Vorrichtung (10, 20, 30, 40, 50) zur Bereitstellung von Unterdruck für die Unterdrucktherapie von Wunden am menschlichen oder tierischen Körper, umfassend
i) eine den Unterdruck erzeugende Einrichtung (1) mit einem Einlass (2) zum Ansaugen von Luft und einem Auslass (3) zum Ausblasen von Luft,
ii) einen Anschluss (4) für eine zur Wundstelle führende Saugleitung (18),
iii) eine Unterdruckverbindung (5) zwischen dem Einlass (3) und dem Anschluss (4), so dass eine Unterdruckkommunikation zwischen der den Unterdruck erzeugenden Einrichtung (1) und der zur Wundstelle führenden Saugleitung (18) herstellbar ist,
iv) mindestens einen Filter (6), welcher im intakten Zustand für Flüssigkeiten undurchlässig ist, wobei der Filter (6) in der Unterdruckverbindung (5) angeordnet ist, **dadurch gekennzeichnet, dass** die Vorrichtung (10, 20, 30, 40, 50) mindestens einen Flüssigkeitsindikator (8) zum Anzeigen und/oder Nachweis von Flüssigkeit umfasst, wobei der mindestens eine Flüssigkeitsindikator (8) stromabwärts des Filters (6) angeordnet ist.

2. Vorrichtung (10, 20, 30, 40, 50) nach Anspruch 1, wobei der Flüssigkeitsindikator (8)
- in demjenigen Abschnitt der Unterdruckverbindung (5), welcher sich zwischen dem Filter (6) und dem Einlass (2) befindet, und/oder
- in der den Unterdruck erzeugenden Einrichtung (1), und/oder
- in dem Bereich des Auslasses (3)
vorhanden ist.

3. Vorrichtung (10, 20, 30, 40, 50) nach Anspruch 1, wobei der Flüssigkeitsindikator (8) auf der zur Unterdruck erzeugenden Einrichtung hin zeigenden Oberfläche des Filters (6) angebracht ist.

4. Vorrichtung (10, 20, 30, 40, 50) nach einem oder mehreren der vorangehenden Ansprüche, wobei es sich bei der einen Unterdruck erzeugenden Einrichtung (1) um eine manuell betriebene Saugvorrichtung oder um eine elektrisch antreibbare Pumpe handelt.

5. Vorrichtung (30, 40, 50) nach einem oder mehreren der vorangehenden Ansprüche, weiterhin umfassend einen Flüssigkeitsaufnahmebehälter (9) zur Aufnahme von aus der Wunde abgesaugten Körperflüssigkeiten (25), wobei der Flüssigkeitsaufnahmebehälter (9) in der Unterdruckverbindung (5) zwischen dem Filter (6) und dem Anschluss für die zur Wundstelle führende Saugleitung (18) angeordnet ist und wobei der Flüssigkeitsaufnahmebehälter (9) mindestens einen Zulauf (26) zum Einspeisen der Körperflüssigkeiten und mindestens einen Ausgang (27) zur Beaufschlagung mit Unterdruck aufweist.

6. Vorrichtung (30, 40, 50) nach Anspruch 5, wobei der Flüssigkeitsaufnahmebehälter (9) in seinem Inneren durch Rippen oder Vorsprünge gebildete Bereiche oder miteinander kommunizierende Kammern aufweist, so dass die im Inneren des Flüssigkeitsaufnahmebehälters (9) gesammelten Körperflüssigkeiten (25) bei einer Bewegung des Flüssigkeitsaufnahmebehälters (9) am unkontrollierten Schwappen gehindert werden.

7. Vorrichtung (50) nach einem der vorangehenden Ansprüche 5 oder 6, wobei die Vorrichtung ein erstes Gehäuseteil (31) mit der den Unterdruck erzeugenden Einrichtung und ein zweites Gehäuseteil (32) umfasst, wobei das zweite Gehäuseteil (32) den Flüssigkeitsaufnahmebehälter (9) bildet und wobei das zweite Gehäuseteil (32) an dem ersten Gehäuseteil (31) der Vorrichtung (50) manuell befestigbar und von dem ersten Gehäuseteil (31) der Vorrichtung (50) manuell lösbar ist.

8. Vorrichtung (50) nach Anspruch 7, wobei es sich bei der den Unterdruck erzeugenden Einrichtung um eine elektrisch angetriebene Pumpe (21) handelt und wobei in dem ersten Gehäuseteil neben der den Unterdruck erzeugenden Einrichtung eine elektronische Steuerungseinheit (7) für die Pumpe, eine Stromversorgung (22) und Mittel (23) zum Bedienen der Steuerungseinheit (7) durch einen Benutzer untergebracht sind.

9. Vorrichtung (10, 20, 30, 40, 50) nach einem oder mehreren der vorangehenden Ansprüche, wobei der Flüssigkeitsindikator (8) eine Substanz umfasst, welche nach Flüssigkeitsaufnahme einen Farbumschlag zeigt.

10. Vorrichtung (10, 20, 30, 40, 50) nach einem oder mehreren der vorangehenden Ansprüche, wobei der Flüssigkeitsindikator (8) eine durch Wasser verteilbare Substanz und ein Trägermaterial umfasst, so dass nach Flüssigkeitsaufnahme eine Farbveränderung beobachtbar ist.

11. Vorrichtung (20, 40, 50) nach einem der vorangehenden Ansprüche 9 oder 10, weiterhin umfassend einen elektronischen Farbsensor (19), mittels dem ein Farbumschlag oder eine Farbveränderung an dem Flüssigkeitsindikator (8) nachweisbar ist.

12. Verwendung eines Flüssigkeitsindikators (8) in einer Vorrichtung (10, 20, 30, 40, 50) nach einem oder mehreren der vorangehenden Ansprüche zum Nachweis von Flüssigkeit, welche stromabwärts des Filters (6) eingedrungen ist.

13. Verwendung eines elektronischen Farbsensors (19) in einer Vorrichtung (20, 40, 50) nach Anspruch 11 zum Nachweis von Flüssigkeit, welche stromabwärts des Filters (6) eingedrungen ist.

14. Verwendung nach Anspruch 12 oder 13, wobei der Nachweis von Flüssigkeitseintritt im Rahmen einer Sicherheitsüberprüfung oder Wartung der Vorrichtung (10, 20, 30, 40, 50) durchgeführt wird.
